# FASCICULE DE BREVET EUROPEEN

(11) **EP 0 627 008 B1**
(45) Date de publication et mention de la délivrance du brevet: **31.05.2000**
(21) Numéro de dépôt: 94900857.7
(22) Date de dépôt: 19.11.1993
(51) Int. Cl.: C12P 17/00, C09B 69/10, A61K 7/00, A61K 7/13

(54) **PROCEDE DE PREPARATION DE PIGMENTS MELANIQUES PAR BIOCONVERSION**
VERFAHREN ZUR HERSTELLUNG VON MELANINPIGMENTE DURCH BIOKONVERSION
METHOD FOR THE PREPARATION OF MELANIC PIGMENTS BY BIOCONVERSION

(30) Priorité: 20.11.1992 FR 9214000
(43) Date de publication de la demande: 07.12.1994
(73) Titulaire: L'OREAL, 75008 Paris (FR)
(72) Inventeur: JUNINO, Alex, F-93180 Livry-Gargan (FR); HILAIRE, Pascal, F-37100 Tours (FR); MARTIN, Richard, F-37210 Rochecorbon (FR)
(74) Mandataire: Casalonga, Axel
(86) Numéro de dépôt international: FR9301138
(87) Numéro de publication internationale: WO9412653

(56) Documents cités:
- EP-A- 0 467 767
- LU-A- 83 173
- CHEMICAL ABSTRACTS, vol. 102, no. 5, 04 Février 1985, Columbus, Ohio, US; abrégé no. 43036H, SEILER, G.J. ET AL.: 'Prevalence of phytomelanin in pericarps of sunflower parental lines and wild species.' page 326; XPCA:V.102,N & CROP SCI. vol. 24, no. 6 , 1984 pages 1202 - 1204

## Description

La présente invention concerne un procédé de préparation de pigments mélaniques par bioconversion et les pigments ainsi obtenus.

Il est connu d'utiliser des pigments colorés dans le domaine cosmétique, et il s'agit essentiellement de pigments minéraux ou encore de pigments issus de colorants directs de synthèse et, dans le cas des pigments noirs, de carbone pur. EP 0467 767 décrit des produits à base de particules lamellaires comportant un pigment mélanique.

Il est notamment connu de produire des colorants jaune-brun par oxydation du 5-hydroxyindole. Il est connu par ailleurs, voir notamment EP 0 441 689, de préparer des pigments mélaniques par voie enzymatique, par polymérisation oxydative de dérivés du 5,6-dihydroxyindole tels que le 5,6-diméthoxyindole ou le 5,6-méthylènedioxyindole.

La demanderesse a maintenant découvert que l'on pouvait obtenir, rapidement, des pigments mélaniques par bioconversion de substrats simples, notamment à l'aide de cellules végétales cultivées in vitro de façon usuelle.

En effet, les procédés proposés jusqu'à présent mettent en oeuvre des précurseurs conventionnels de biosynthèse des mélanines qui s'avèrent chers.

Au contraire, selon l'invention, il s'agit de précurseurs et de composés biologiques courants, au prix modique et d'emploi facile. Le mécanisme de bioconversion enzymatique selon l'invention ne met pas en oeuvre de polyphénoloxydases.

Ainsi, la présente invention concerne un procédé de préparation de pigments mélaniques par bioconversion enzymatique, mettant en oeuvre un substrat précurseur de mélanine et des cellules végétales. Selon l'invention :
a) des cellules végétales de pavot (Papaver somniferum) préalablement cultivées sont séparées de leur milieu de culture et repiquées dans un milieu de culture à une concentration allant de 10 à 100 g/l,
b) on broye les cellules au moins partiellement, avant ou à la fin du temps de latence,
c) on met en présence les cellules au moins partiellement broyées, dans un milieu de bioconversion, avec un substrat précurseur de mélanine choisi parmi l'indole, l'indoline, la dihydroxyphénylalanine, la tyramine et la tyrosine,
d) on récupère le pigment mélanique précipité.

Dans une première étape, des cellules indifférenciées de pavot sont cultivées in vitro dans un milieu de culture usuel. Après un temps de croissance suffisant, les cellules sont séparées de leur milieu, par exemple par filtration. La durée du temps de croissance des cellules est sans importance sur le procédé et dépend notamment de la quantité de cellules que l'on souhaite obtenir.

Comme milieu de culture usuel, on peut utiliser le milieu de Heller. Ce milieu peut éventuellement contenir en outre des vitamines et/ou des hormones.

Par cellules indifférenciées de pavot, on entend des cultures cellulaires issues de fragments de plante entière, décontaminées et placées sur des milieux de culture solides choisis de telle sorte que les cellules s'y multiplient sans donner naissance à des formes tissulaires organisées. Ces cellules sont ensuite transférées dans des milieux liquides où leur croissance s'effectue par simple multiplication cellulaire. Ces cultures sont comparables à celles de bactéries.

Dans une seconde étape, les cellules obtenues sont repiquées dans un milieu de culture usuel, à une concentration en matière fraîche cellulaire de 10 à 100 g/l. Comme milieu de culture pour le repiquage, on utilise un milieu de culture neuf On peut utiliser pour le repiquage également un milieu de Heller, contenant éventuellement des vitamines et/ou des hormones. Les cellules sont maintenues dans ce milieu pendant une durée inférieure ou égale au temps de latence.

Le temps de latence est le temps nécessaire à une cellule fraîchement repiquée pour s'adapter au milieu et durant lequel la concentration en cellule reste constante avant d'y croître. Lors de prélèvements périodiques, on peut ainsi constater que la concentration en cellule reste sensiblement identique à la concentration initiale - les cellules sont dans la période de latence - puis la concentration croît significativement lorsque le temps de latence est terminé; les cellules sont alors passées à l'étape de croissance. Selon la souche utilisée, ce temps de latence peut varier entre 6 heures et 7 jours.

On préfère repiquer les cellules à une concentration en matière fraîche cellulaire de 20 à 50 g/l.

Dans une troisième étape, avant ou à la fin du temps de latence, les cellules sont de préférence broyées pour obtenir un broyat cellulaire:

Le broyage cellulaire peut s'effectuer dans le milieu de culture lui-même, mais on préfère, auparavant, isoler les cellules, par filtration, par exemple. Pour obtenir le broyat cellulaire, on peut utiliser des moyens usuels comme un broyeur Potter ou Ultra Turrax. De préférence, le broyage a lieu à basse température, et en particulier entre 0 et 15°C.

Dans une quatrième étape, pour réaliser la bioconversion, le broyat cellulaire est mis en contact avec le substrat précurseur de mélanine choisi parmi l'indole, l'indoline, la dihydroxyphénylalanine (DOPA), la tyramine et la tyrosine. De préféfence, la mise en présence a lieu à une température comprise entre 10 et 70°C.

Le milieu de bioconversion peut être constitué par le broyat cellulaire. On peut aussi reprendre le broyat cellulaire dans de l'eau déminéralisée ou dans une solution tamponnée. Dans ce cas, le pH est de préférence choisi entre 3 et 9.

La réaction s'effectue en mettant en présence le substrat et le broyat cellulaire, de préférence dans un milieu tamponné, la concentration en substrat pouvant varier dans de larges limites et étant fonction de sa solubilité dans le milieu aqueux.

La réaction est de préférence mise en oeuvre sous agitation.

La concentration en substrat est généralement comprise entre 10 mg et 2 g par litre de solution tamponnée contenant de 20 à 200 g de matière fraîche cellulaire. On peut augmenter la concentration en substrat en dissolvant celui-ci dans un solvant organique miscible à l'eau, tel que l'éthanol.

Dans cette étape de bioconversion, le temps de réaction est variable et dépend notamment du substrat, de la température de réaction et du pH du milieu.

De façon générale, un précipité noir apparaît après 30 minutes à 24 heures. On peut récupérer le pigment précipité par décantation, filtration ou centrifugation ou tout autre moyen de séparation adapté. On peut récupérer le filtrat et recommencer l'opération en y ajoutant du substrat, dans les conditions décrites ci-dessus. De préférence, on filtre sur crible dont les mailles ont une dimension inférieure à 5 µm, afin notamment de débarrasser les pigments des résidus protéiques.

L'analyse de ce pigment par RPE (résonance paramagnétique électronique) effectuée à l'aide d'un spectromètre ER 200 D BRUCKER à 9,52 GHz avec une fréquence de modulation de champ de 100 KHz et une puissance de micro-onde de 1,9 mw, révèle une absorption maximale vers 3470 Gauss, caractéristique des mélanines.

Le pigment mélanique obtenu peut ensuite être lavé à l'éthanol puis à l'eau afin d'éliminer le précurseur de mélanine.

Les pigments obtenus, éventuellement lavés et séchés, sont particulièrement utiles dans le domaine de la cosmétique, pour la préparation de produits de maquillage, de composition solaire ou encore de composition de coloration capillaire.

Ils peuvent être introduits en vue de ces applications dans un milieu cosmétiquement acceptable à base d'eau et/ou de mélange eau-solvant organique ou d'un ou plusieurs solvant(s), et éventuellement comportant encore d'autres additifs usuels en cosmétique, par exemple des agents tensio-actifs, des épaississants, des conservateurs.

Selon une variante de l'invention, le pigment mélanique peut être déposé sur une charge minérale constituée de particules inertes, ayant une granulométrie usuelle pour ces supports, par exemple une granulométrie inférieure à 20 µm, et de préférence 10 µm.

De façon connue, le pigment peut être déposé et/ou absorbé sur des particules minérales sous forme lamellaire ou non lamellaire, organiques lamellaires ou non lamellaires, colorées ou non. Ces particules ont une granulométrie moyenne comprise entre 0,01 et 200 µm.

On obtient ainsi des poudres colorées pouvant être utilisées en cosmétique.

Cette poudre colorée peut être préparée en dispersant les particules minérales ou organiques dans la solution de précurseur mélanique et de broyat cellulaire où a lieu la bioconversion ci-dessus. La poudre contenant le pigment est séparée, par exemple par filtration, après le temps de réaction nécessaire à la formation des pigments mélaniques, et on la lave à l'eau et on la sèche.

Ces poudres peuvent également être préparées par absorption du pigment mélanique préparé selon l'invention sur et/ou dans les particules minérales ou organiques définies ci-dessus.

Dans cette variante, on peut procéder en dispersant le pigment mélanique préalablement formé dans un milieu où les particules sont insolubles et contenant lesdites particules, et, après absorption du pigment, on sèche les particules pigmentées.

On peut utiliser, comme charges minérales, des particules de carbonate de calcium, de silice ou d'oxyde de titane ayant la granulométrie définie ci-dessus.

Comme charges organiques, on utilise de préférence des particules de polymères dérivés de la kératine, éventuellement modifiées, de polymères dérivés de la chitine, éventuellement désacétylés, de fibroïne de soie, de polymères synthétiques choisis parmi le polyméthacrylate de méthyle réticulé, la poly-β-alanine réticulée, des microsphères creuses de copolymères chlorure de vinylidène et acrylonitrile ou encore des microsphères poreuses de polyamide-12, polyamide-6 ou de copolyamide-6/12, ainsi que des poudres de silicone constituées par des gommes, des résines, des élastomères d'organopolysiloxane.

Ces particules ont une granulométrie de préférence supérieure à 0,1µm.

Les particules lamellaires sont des particules minérales ou organiques se présentant sous forme de feuillets, éventuellement stratifiés. Ces feuillets se caractérisent par une épaisseur plus faible que la plus grande dimension. De préférence, le rapport entre la plus grande dimension et l'épaisseur est compris entre 2 et 100. La dimension la plus grande est généralement inférieure à 100 µm.

Les exemples qui suivent sont destinés à illustrer l'invention sans pour autant présenter un caractère limitatif.

### Exemples de préparation d'un pigment mélanique à partir d'indole.

On utilise différentes souches issues de cultures indifférenciées de pavot, ces souches ayant été reprises en milieu liquide à partir de cals sur gélose, et l'on réalise les étapes suivates.

### 1ère étape

Les cellules indifférenciées de pavot sont cultivées in vitro à 26°C dans les milieux suivants définis ci-après :

| MILIEU 1 | | |
|---|---|---|
| KCl | 0,750 g/l | Macro-éléments de Heller |
| NaNO₃ | 0,600 g/l | Macro-éléments de Heller |
| MgSO₄,7H₂O | 0,250 g/l | Macro-éléments de Heller |
| NaH₂PO₄,2H₂O | 0,141 g/l | Macro-éléments de Heller |
| CaCl₂,2H₂O | 0,075 g/l | Macro-éléments de Heller |
| ZnSO₄,7H₂O | 1 mg/l | Micro-éléments de Heller |
| H₃BO₃ | 1 mg/l | Micro-éléments de Heller |
| MnSO₄,1H₂O | 0,076 mg/l | Micro-éléments de Heller |
| CuSO₄,5H₂O | 0,03 mg/l | Micro-éléments de Heller |
| AlCl₃,6H₂O | 0,05 mg/l | Micro-éléments de Heller |
| Kl | 0,01 mg/l | Micro-éléments de Heller |
| NiCl₂,6H₂O | 0,03 mg/l | Micro-éléments de Heller |
| FeCl₃,6H₂O | 1,00 mg/l | |
| Vitamines de Morel | 2 ml | |
| Acide 2,4-dichlorophénoxyacétique 10⁻⁴ M | 1 ml | |
| Kinétine 10⁻³ M | 1 ml | |
| Glucose | 30 g/l | |

| MILIEU 2 | | |
|---|---|---|
| KCl | 0,750 g/l | Macro-éléments de Heller |
| NaNO₃ | 0,600 g/l | Macro-éléments de Heller |
| MgSO₄,7H₂O | 0,250 g/l | Macro-éléments de Heller |
| NaH₂PO₄,2H₂O | 0,141 g/l | Macro-éléments de Heller |
| CaCl₂,2H₂O | 0,075 g/l | Macro-éléments de Heller |
| ZnSO₄,7H₂O | 1 mg/l | Micro-éléments de Heller |
| H₃BO₃ | 1 mg/l | Micro-éléments de Heller |
| MnSO₄,1H₂O | 0,076 mg/l | Micro-éléments de Heller |
| CuSO₄,5H₂O | 0,03 mg/l | Micro-éléments de Heller |
| AlCl₃,6H₂O | 0,05 mg/l | Micro-éléments de Heller |
| Kl | 0,01 mg/l | Micro-éléments de Heller |
| NiCl₂,6H₂O | 0,03 mg/l | Micro-éléments de Heller |
| FeCl₃,6H₂O | 1,00 mg/l | |
| Glucose | 30 g/l | |

| MILIEU 3 | | |
|---|---|---|
| KCl | 1,500 g/l | |
| KNO₃ | 1,450 g/l | |
| (NH₄)₂SO₄ | 0,134 g/l | |
| KH₂PO₄ | 0,150 g/l | |
| MgSO₄,7H₂O | 0,500 g/l | |
| CaCl₂,2H₂O | 0,150 g/l | |
| ZnSO₄,7H₂O | 1 mg/l | Micro-éléments de Heller |
| H₃BO₃ | 1 mg/l | Micro-éléments de Heller |
| MnSO₄,1H₂O | 0,076 mg/l | Micro-éléments de Heller |
| CuSO₄,5H₂O | 0,03 mg/l | Micro-éléments de Heller |
| AlCl₃,6H₂O | 0,05 mg/l | Micro-éléments de Heller |
| Kl | 0,01 mg/l | Micro-éléments de Heller |
| NiCl₂,6H₂O | 0,03 mg/l | Micro-éléments de Heller |
| FeCl₃,6H₂O | 1,00 mg/l | |
| Vitamines de Morel | 2 ml | |
| Acide 2,4-dichlorophénoxyacétique 10⁻⁴ M | 1 ml | |
| Kinétine 10⁻³ M | 1 ml | |
| Glucose | 30 g/l | |

Par vitamines de Morel, on entend un mélange comportant, pour 100 ml :

| | |
|---|---|
| Panthoténate de Ca | 0,050 g |
| Acide nicotinique | 0,050 g |
| Méso inositol | 5,000 g |
| Pyridoxine (B6) | 0,050 g |
| Thiamine (B1) | 0,050 g |
| Biotine | 0,5 mg |

Sur huit souches testées, quatre ont été cultivées dans le milieu 1, trois dans le milieu 2 et une dans le milieu 3.

Après un temps de croisance permettant à la concentration en cellules d'atteindre 400 g/l du milieu, on récupère les cellules par filtration sur 50 µm.

### 2ème étape

Les cellules obtenues à la première étape sont repiquées dans un milieu de culture identique à 4°C à raison de 50 g/l. Après un temps de latence variant entre 1 à 2 jours, les cellules sont filtrées.

### 3ème étape

Les cellules sont broyées à 4°C dans un Potter. Le broyat est repris dans un milieu tamponné de pH compris entre 6,5 et 7, à raison d'environ 1 g de cellule pour 10 ml de solution tampon.

### 4ème étape

On ajoute 50 mg d'indole par litre de solution obtenue à la troisième étape, sous agitation et à température ambiante. Dès les quinze premières minutes, une coloration rose apparaît, cette coloration devient mauve pour se terminer par du noir au bout de deux heures. On filtre alors cette suspension sur un crible dont les mailles ont une dimension inférieure à 5 microns. Le précipité est ensuite lavé à l'eau puis séché.

A partir des huit souches testées après culture dans chacun des trois milieux décrits, des pigments ont été obtenus alors que ces trois milieux de culture ne donnent aucune réaction avec le même précurseur, que ce soit avant ou après la culture des cellules.

### Analyse RPE des pigments obtenus :

Le spectre RPE révèle une absorption maximum à 3470 Gauss.

### EXEMPLES D'APPLICATION

| EXEMPLE A : MASCARA CREME | |
|---|---|
| - Pigment noir obtenu selon l'exemple 1 | 15 g |
| - Stéarate de triéthanolamine | 15 g |
| - Cire de Candellila | 8 g |
| - Cire de Carnauba | 10 g |
| - Hydroxyéthylcellulose | 0,9 g |
| - Hydrolysat de kératine (exprimé en matière sèche) | 0,75 g |
| - Conservateurs qs | |
| - Eau | qsp 100 g |

| EXEMPLE B : GEL CAPILLAIRE | |
|---|---|
| - Pigment noir obtenu selon l'exemple 1 | 0,5 g |
| - Copolymère de vinylpyrrolidone/acétate de vinyle, vendu sous la dénomination "PVP/VA S 630" par la Société GAF | 1,5 g |
| - Alcool éthylique | 15 g |
| - "Carbopol 940" (GOODRICH CHEMICAL) | 0,7 g |
| - Triéthanolamine qs pH = 7,5 | |
| - Conservateurs qs | |
| - Eau | qsp 100 g |

## Revendications

1. Procédé de préparation d'un pigment mélanique par bioconversion enzymatique, mettant en oeuvre un substrat précurseur de mélanine et des cellules végétales, caractérisé en ce que :
a) des cellules végétales de pavot (Papaver somniferum) préalablement cultivées sont séparées de leur milieu de culture et repiquées dans un milieu de culture à une concentration allant de 10 à 100 g/l,
b) on broye les cellules au moins partiellement, avant ou à la fin du temps de latence,
c) on met en présence les cellules au moins partiellement broyées, dans un milieu de bioconversion, avec un substrat précurseur de mélanine choisi parmi l'indole, l'indoline, la dihydroxyphénylalanine, la tyramine et la tyrosine,
d) on récupère le pigment mélanique précipité.

2. Procédé selon la revendication 1, caractérisé en ce que le milieu de culture où l'on repique les cellules préalablement cultivées, a une concentration comprise entre 20 et 50 g/l.

3. Procédé selon la revendication 1 ou 2, caractérisé en ce que le substrat est mis en présence avec un broyat cellulaire.

4. Procédé selon la revendication 3, caractérisé en ce que le broyat cellulaire est obtenu par broyage des cellules préalablement séparées de leur milieu de culture, à une température comprise entre 0 et 15°C.

5. Procédé selon l'une des revendications 1 à 4, caractérisé en ce que la mise en présence des cellules au moins partiellement broyées avec le substrat est mise en oeuvre sous agitation.

6. Procédé selon l'une des revendications 1 à 5, caractérisé en ce que la mise en présence des cellules au moins partiellement broyées avec le substrat a lieu à une température comprise entre 10 et 70°C.

7. Procédé selon l'une des revendications 1 à 6, caractérisé en ce que la mise en présence desdites cellules avec ledit substrat est réalisée dans une solution tamponnée dont le pH varie entre 3 et 9.

8. Procédé selon l'une des revendications 1 à 7, caractérisé en ce que l'on récupère le pigment précipité par filtration, décantation ou centrifugation.

9. Procédé selon la revendication 8, caractérisé en ce que l'on récupère le pigment précipité par filtration du milieu de bioconversion sur un crible dont les mailles ont une dimension inférieure à 5 µm.

## Claims

1. Process for preparing a melanin pigment by enzymatic bioconversion, employing a melanin precursor substrate and plant cells, characterized in that:
a) poppy (Papaver somniferum) plant cells previously cultured, are separated from their culture medium and subcultured in a culture medium at a concentration ranging from 10 to 100 g/l,
b) the cells are at least partially ground before or at the end of the latency time,
c) the at least partially ground cells are brought into contact, in a bioconversion medium, with a melanin precursor substrate chosen from indole, indoline, dihydroxyphenylalanine, tyramine and tyrosine,
d) the precipitated melanin pigment is recovered.

2. Process according to claim 1, characterized in that the culture medium in which the previously cultured cells are subcultured, has a concentration of between 20 and 50 g/l.

3. Process according to claim 1 or 2, characterized in that the substrate is brought into contact with a ground cell preparation.

4. Process according to claim 3, characterized in that the ground cell preparation is obtained by grinding the cells, previously separated from their culture medium, at a temperature of between 0 and 15°C.

5. Process according to one of claims 1 to 4, characterized in that the contacting of the at least partially ground cells with the substrate is carried out with stirring.

6. Process according to one of claims 1 to 5, characterized in that the contacting of the at least partially ground cells with the substrate takes place at a temperature of between 10 and 70°C.

7. Process according to one of claims 1 to 6, characterized in that the contacting of the said cells with the said substrate is carried out in a buffered solution whose pH varies between 3 and 9.

8. Process according to one of claims 1 to 7, characterized in that the precipitated pigment is recovered by filtration, decantation or centrifugation.

9. Process according to claim 8, characterized in that the precipitated pigment is recovered by filtration of the bioconversion medium through a screen of mesh size less than 5 µm.

## Patentansprüche

1. Verfahren zur Herstellung eines Melaninpigments durch enzymatische Biokonversion unter Verwendung eines Precursors von Melanin als Substrat und unter Verwendung von Pflanzenzellen, dadurch gekennzeichnet, daß
a) Pflanzenzellen von Mohn (Papaver somniferum), die vorab kultiviert wurden, aus ihrem Kulturmedium entfernt und in einer Konzentration im Bereich von 10 bis 100 g/l in einem Kulturmedium subkultiviert werden,
b) die Zellen in der Latenzzeit oder am Ende der Latenzzeit zumindest teilweise zerkleinert werden,
c) die zumindest zum Teil zerkleinerten Zellen in einem Medium für die Biokonversion mit einem als Substrat dienenden Melaninprecursor, der unter Indol, Indolin, Dihydroxyphenylalanin, Tyramin und Tyrosin ausgewählt ist, in Kontakt gebracht werden, und
d) das ausgefallene Melaninpigment gewonnen wird.

2. Verfahren nach Anspruch 1, dadurch gekennzeichnet, daß das Kulturmedium, in dem die zuvor kultivierten Zellen subkultiviert werden, eine Konzentration von 20 bis 50 g/l aufweist.

3. Verfahren nach Anspruch 1 oder 2, dadurch gekennzeichnet, daß das Substrat mit einer zerkleinerten Zellmasse in Kontakt gebracht wird.

4. Verfahren nach Anspruch 3, dadurch gekennzeichnet, daß die zerkleinerte Zellmasse durch Zerkleinern von Zellen, die zuvor von dem Kulturmedium abgetrennt wurden, bei einer Temperatur im Bereich von 0 bis 15 °C hergestellt wird.

5. Verfahren nach einem der Ansprüche 1 bis 4, dadurch gekennzeichnet, daß die zumindest zum Teil zerkleinerten Zellen mit dem Substrat unter Rühren in Kontakt gebracht werden.

6. Verfahren nach einem der Ansprüche 1 bis 5, dadurch gekennzeichnet, daß die zumindest zum Teil zerkleinerten Zellen mit dem Substrat bei einer Temperatur im Bereich von 10 bis 70 °C in Kontakt gebracht werden.

7. Verfahren nach einem der Ansprüche 1 bis 6, dadurch gekennzeichnet, daß die Zellen mit dem Substrat in einer Pufferlösung, deren pH-Wert im Bereich von 3 bis 9 liegt, in Kontakt gebracht werden.

8. Verfahren nach einem der Ansprüche 1 bis 7, dadurch gekennzeichnet, daß das Pigment durch Filtrieren, Dekantieren oder Zentrifugieren gewonnen wird.

9. Verfahren nach Anspruch 8, dadurch gekennzeichnet, daß das ausgefallene Pigment gewonnen wird, indem das Biokonversionsmedium an einem Sieb filtriert wird, dessen Maschenweite unter 5 µm liegt.
